# EUROPEAN PATENT SPECIFICATION

(11) **EP 3 123 372 B1**
(45) Date of publication and mention of the grant of the patent: **12.12.2018**
(21) Application number: 15716916.0
(22) Date of filing: 20.03.2015
(51) Int. Cl.: G06F 19/00

(54) **DETERMINING A LEVEL OF HYPOGLYCEMIC UNAWARENESS DISPLAYED BY A PATIENT**
BESTIMMUNG DES VON EINEM PATIENTEN ANGEZEIGTEN GRADES AN HYPOGLYKÄMIE-WAHRNEHMUNGSSTÖRUNG
DÉTERMINATION DE NIVEAU D'INSENSIBILITÉ À L'HYPOGLYCÉMIE AFFICHÉE PAR UN PATIENT

(30) Priority: 28.03.2014 US 201461971694 P
(43) Date of publication of application: 01.02.2017
(73) Proprietor: Joslin Diabetes Center, Inc., Boston, MA 02115 (US)
(72) Inventor: WOLPERT, Howard Allan, Brookline, Massachusetts 02446 (US)
(74) Representative: Brown, Alexander Edward
(86) International application number: PCT/US2015/021811
(87) International publication number: WO 2015/148313

(56) References cited:
- WO-A1-2013/108122
- D. COX ET AL: "Blood Glucose Awareness Training Delivered Over the Internet", DIABETES CARE, vol. 31, no. 8, 13 May 2008 (2008-05-13), pages 1527-1528, XP055196278, ISSN: 0149-5992, DOI: 10.2337/dc07-1956
- D. J. COX ET AL: "Blood Glucose Awareness Training: What Is It, Where Is It, and Where Is It Going?", DIABETES SPECTRUM, vol. 19, no. 1, 1 January 2006 (2006-01-01), pages 43-49, XP055196290, ISSN: 1040-9165, DOI: 10.2337/diaspect.19.1.43
- BROERS ET AL: "Blood glucose awareness training in Dutch type 1 diabetes patients: one-year follow-up", THE NETHERLANDS JOURNAL OF MEDICINE, vol. 63, no. 5, 1 May 2005 (2005-05-01), pages 164-169, XP055196298,
- CLARKE W L ET AL: "Reduced awareness of hypoglycemia in adults with IDDM. A prospective study of hypoglycemic frequency and associated symptoms", DIABETES CARE, AMERICAN DIABETES ASSOCIATION, ALEXANDRIA, VA, US, vol. 18, no. 4, 1 April 1995 (1995-04-01), pages 517-522, XP008176692, ISSN: 0149-5992, DOI: 10.2337/DIACARE.18.4.517
- GOLD A E ET AL: "Frequency of severe hypoglycemia in patients with type I diabetes with impaired awareness of hypoglycemia", DIABETES CARE, AMERICAN DIABETES ASSOCIATION, ALEXANDRIA, VA, US, vol. 17, no. 7, 1 July 1994 (1994-07-01), pages 697-703, XP008176691, ISSN: 0149-5992, DOI: 10.2337/DIACARE.17.7.697
- ULRIK PEDERSEN-BJERGAARD ET AL: "Recall of severe hypoglycaemia and self-estimated state of awareness in type 1 diabetes", DIABETES/METABOLISM RESEARCH AND REVIEWS, vol. 19, no. 3, 1 January 2003 (2003-01-01), pages 232-240, XP055196268, ISSN: 1520-7552, DOI: 10.1002/dmrr.377

## Description

### BACKGROUND

Hypoglycemia is defined as a blood glucose level below 70 mg/dl. Hypoglycemia unawareness is a complication of diabetes in which the patient is unaware of a decline in the blood glucose concentration because it fails to activate the autonomic nervous system which generates the characteristic symptoms of hypoglycemia (such as palpitations, sweating, anxiety) that serve to warn the patient of the dropping blood glucose. Typically, patients who are aware of their symptoms can take action and eat to help reverse the hypoglycemia.

However, hypoglycemia unawareness can result in prolonged exposure to hypoglycemia putting the patient at risk for a number of severe hypoglycemia related complications, including but not limited to: seizures, loss of consciousness, or brain damage. The development of hypoglycemia unawareness also makes the control of the patient's blood glucose levels more difficult.

As such, novel methods for detecting hypo-awareness and providing methods for intervention before it results in severe complications are of the utmost importance for diabetic patients and the healthcare community.

A system for determining the level of hypoglycemia unawareness by calculating the difference in-between the patient estimate and the actual measured blood glucose level is BGATHome, presented in D. COX ET AL: "Blood Glucose Awareness Training Delivered Over the Internet", DIABETES CARE, vol. 31, no. 8, 13 May 2008 (2008-05-13), pages 1527-1528, XP055196278, ISSN: 0149-5992, DOI: 10.2337/dc07-1956, and D. J. COX ET AL: "Blood Glucose Awareness Training: What Is It, Where Is It, and Where Is It Going?",DIABETES SPECTRUM, vol. 19, no. 1, 1 January 2006 (2006-01-01), pages 43-49, XP055196290, ISSN: 1040-9165, DOI: 10.2337/diaspect.19.1.43.

### SUMMARY

According to the invention there are provided a method, apparatus and a carrier carrying computer program code according to claims 1, 8 and 15. Preferable features of the invention are defined in the dependent claims.

### BRIEF DESCRIPTION OF THE OF DRAWINGS

The foregoing and other objects, features and advantages will be apparent from the following more particular description of the embodiments, as illustrated in the accompanying drawings in which like reference characters refer to the same parts throughout the different views. The drawings are not necessarily to scale, emphasis instead being placed upon illustrating the principles of the embodiments.
FIG. 1 is a block diagram illustrative of a hypoglycemic unawareness identification environment for determining a level of hypoglycemic unawareness displayed by a patient.
FIG. 2 is a high-level block diagram of an exemplary hypo-unawareness detection and treatment system and processor (HUDTS).
FIG. 3 is a flow diagram of a method for determining a level of hypoglycemic unawareness displayed by a patient, in accordance with an example embodiment of the present disclosure.
FIG. 4 is a block diagram illustrative of a hypoglycemic unawareness identification environment illustrating a process for determining a patient's level of hypo-unawareness, in accordance with an example embodiment of the present disclosure.
FIG. 5 is a diagram illustrating a hypo-report, in accordance with an example embodiment of the present disclosure.
FIG. 6 is a block diagram illustrative of a hypoglycemic unawareness identification environment illustrating a process for issuing a second query based on patient responses to a first query, in accordance with an example embodiment of the present disclosure.
FIG. 7 is a block diagram illustrative of a hypoglycemic unawareness identification environment illustrating a process for providing a clinician with treatment options, in accordance with an example embodiment of the present disclosure.

### DETAILED DESCRIPTION

Low blood glucose or hypoglycemia is one of the most common problems associated with diabetes. Hypoglycemia is defined as a blood glucose level below 70 mg/dl as measured by a glucose measuring device that tests blood or plasma glucose. Hypoglycemia is usually unpleasant, with the most common symptoms including feeling shaky, sweaty, and having one's heart pound. The most common reasons for hypoglycemia include too much insulin, too little food, or too much activity, or a combination thereof. Most hypoglycemia is mild with recognizable symptoms, and if quickly and appropriately treated it is more of an inconvenience than a cause for alarm.

Severe hypoglycemia, however, is defined as the point when a patient with diabetes is not able to independently treat his or herself. The most common reasons for severe hypoglycemia are failure to recognize symptoms or delays in the treatment. Medical research indicates that the typical physical and emotional symptoms of a low blood glucose become attenuated with increasing duration of diabetes. The symptoms may become too subtle for a patient with diabetes to recognize or develop only at a glucose concentration that is so low that the patient with diabetes cannot appropriately react to them. This condition is called hypoglycemic unawareness. Experts believe hypoglycemic unawareness may be due to nerve damage (neuropathy) and/or recurrent hypoglycemia.

Embodiments of the present disclosure relate to methods, systems, apparatus, or computer readable medium, with program codes embodied thereon, for determining a level of hypoglycemic unawareness displayed by a patient.

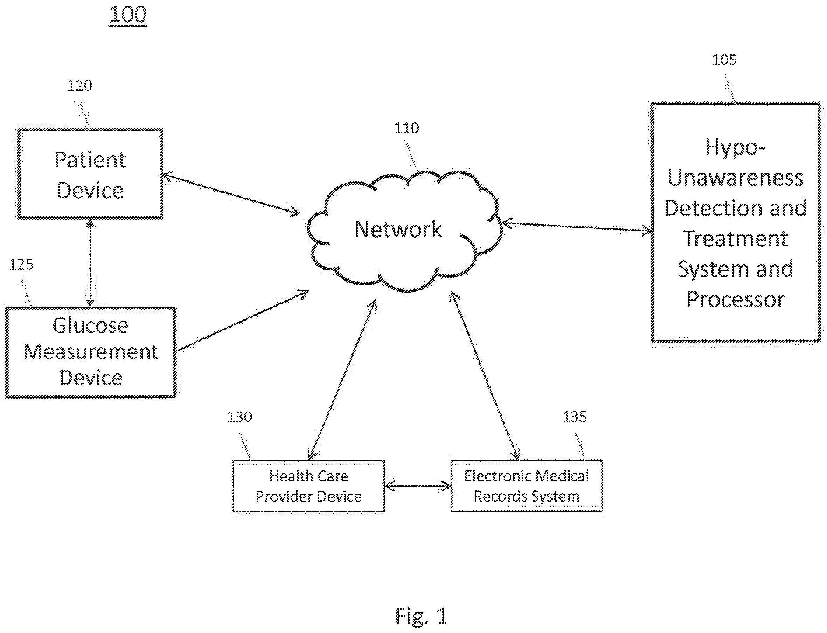

FIG. 1 is a block diagram illustrative of a hypoglycemic unawareness identification environment 100 for determining a level of hypoglycemic unawareness displayed by a patient. As illustrated by FIG. 1, the environment includes a hypo-unawareness detection and treatment system and processor (HUDTS) 105, network 110, patient device 120, glucose measurement device 125, health care provider device 130, and electronic medical records (EMR) system 135.

The HUDTS 105 is in communication with the patient device 120, glucose measurement device 125, health care provider device 120 and EMR system 135 via the network 110. The network 110 can be a public network that may comprise an unsecured wide-area network (WAN), such as the Internet, a wireless network, a local-area network, or another type of network. In other embodiments, the network 110 can be a protected network that includes a secured computer network such as a local-area network (LAN) in an office or a data center. The LAN may be a corporate network including a plurality of work stations (not shown). The plurality of work stations can be operatively coupled to a database (not shown), FTP (file transfer protocol) server (not shown), and intranet server (not shown).

The patient device 120 can be a "wired or wireless transmit/receive unit" (WTRU). The telTI1 WTRU includes but is not limited to a user equipment (UE), a mobile station, a fixed or mobile subscriber unit, a pager, a cellular telephone, a personal digital assistant (PDA), a computer, or any other type of device capable of operating in a communications network. The patient device 120 is in communication with the glucose measurement device 125. The glucose measurement device 125 can be any medical device used to determine the patient's glucose concentration.

In operation, a patient utilizes the glucose measurement device 125 to measure the patient's glucose concentration and report the glucose concentration to the HUDTS 105. In an example embodiment, the glucose measurement device 125 is in direct communication with the HUDTS 105 via network 110 and directly reports the patient's glucose concentration to the HUDTS 105. In other example embodiments, the patient's glucose concentration, which is in communication with the patient device 120, is reported to the HUDTS 105 via the patient device 120. In yet another example, the patient may manually report the patient's glucose concentration to the HUDTS via a communication interface on the patient device 120.

In response to receiving the patient's glucose level, the HUDTS 105 determines a query to send to the patient device 120. The query can include several prompts that require a response from the patient. Once the HUDTS 105 receives the responses to the prompts, the HUDTS 105 determines a level of hypoglycemic unawareness of the patient based at least upon the patient response. In some examples, the HUDTS 105 learns from the patient's previous responses and sends a query included specific prompts tailored for the patient. In another example, the HUDTS 105 can send a query with a reduced number of prompts based on the HUDTS 105 recording a history of the patient's responses.

In response to determining the patient's level of hypoglycemic unawareness, the HUDTS 105 can generate a report for use by a clinician. In addition to reporting the patient's level of hypoglycemic unawareness, the report can include therapeutic recommendations for use by the clinician. The report can be sent to the health care device 130 of the clinician via network 110. In addition, the report can be stored in the EMR system 135 for later retrieval by the clinician, patient, or any other person authorized to access the patient's medical information.

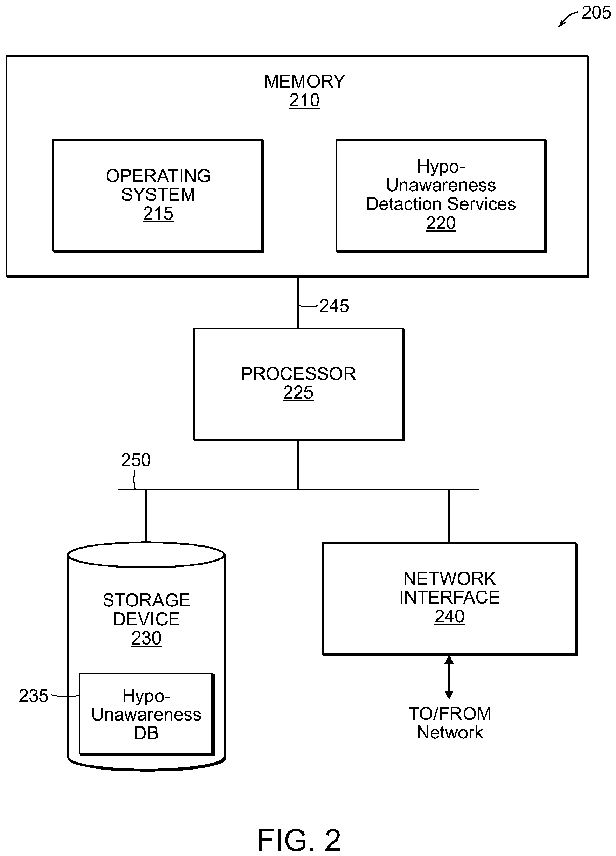

FIG. 2 is a high-level block diagram of an exemplary hypo-unawareness detection and treatment system and processor (HUDTS) 205 that may be used with embodiments described herein. HUDTS 205 comprises a memory 210 coupled to processor(s) 225 via a memory bus 245 and, a storage device 230 and a network interface 240 coupled to the processor 225 via an input/output (I/O) bus 250. It should be noted that the HUDTS 205 may include other devices, such as keyboards, display units and the like. The network interface 240 interfaces the HUDTS 205 with a communications network (e.g., network 110 of FIG. 1), and enables data (*e.g*., packets) to be transferred between the HUDTS 205 and other devices in communication with the communications network. To that end, network interface 240 comprises conventional circuitry that incorporates signal, electrical and mechanical characteristics, and interchange circuits, needed to interface with the physical media of the communications network and protocols running over that media.

The memory 210 is a non-transitory computer-readable medium implemented as a RAM comprising RAM devices, such as DRAM devices and/or flash memory devices. Memory (i.e., data storage device) 210 contains various software and data structures used by the processor 225 including software and data structures that implement aspects of the embodiments described herein. Specifically, memory 210 includes an operating system 215 and hypo-unawareness detection services 220. The operating system 215 functionally organizes the HUDTS 205 by invoking operations in support of software processes and services executing on the HUDTS 205, such as hypo-unawareness detection services 220. Hypo-unawareness detection services 220, as will be described below, comprises computer-executable instructions to determine a level of hypoglycemic unawareness displayed by a patient.

Storage device 230 is a conventional storage device (e.g., disk) that comprises hypo-unawareness database (DB) 235 which is a data structure that is configured to hold various information used to correlate a measured glucose concentration with one or more prompts to be issued to a patient with diabetes associated with the measured glucose concentrations. In addition, the data structure is configured to hold various information used to correlate the patient's responses to the one or more prompts with a level of hypoglycemic unawareness.

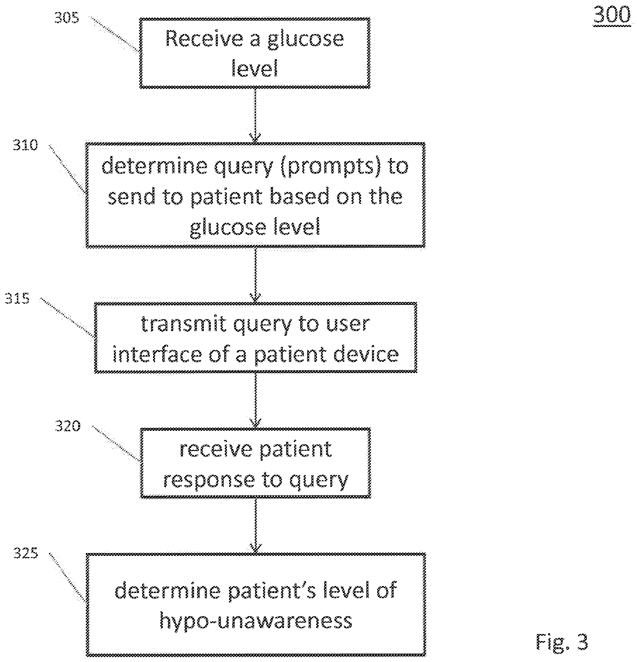

FIG. 3 is a flow diagram of a method 300 for determining a level of hypoglycemic unawareness displayed by a patient. The method 300 can be implemented by, for example, a hypo-unawareness detection and treatment system and processor (HUDTS) (e.g., the HUDTS 105 of FIG. 1). At 305, the method 300 includes receiving a glucose concentration (e.g., by the processor(s) 225 of FIG. 2). The method 300, at 315, includes determining, by the one or more processors, a query based upon the received glucose concentration and a data structure. The query includes at least one prompt that is selected using the data structure, which is stored in a data storage device (e.g., the storage device 230 of FIG. 2). The data structure enables the processor to select at least one prompt because it correlates one or more subject prompts to glucose concentrations.

At 315, the method 300 includes transmitting the query to a user interface of a patient device (e.g., patient device 120 of FIG. 1). The user interface is a graphical interface displayed in a display of the patient device. The user interface enables a patient to communicate with the HUDTS via prompts in the query that are displayed to the patient. At 320, the method 300 includes receiving the patient's responses to the prompts in the query. Based on the patient's response, the method, at 325, includes determining a level of hypoglycemic unawareness of the patient.

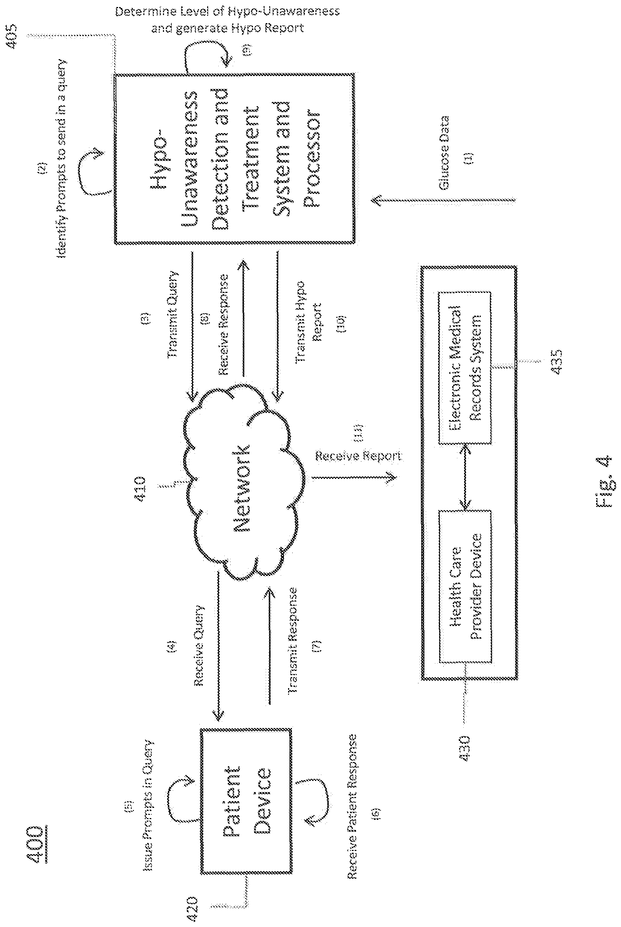

With reference now to FIGS. 4-7, the interaction between various components of a hypoglycemic unawareness identification environment (e.g., the 100 hypoglycemic unawareness identification environment of FIG. 1) is illustrated. For purposes of the example, however, the illustration has been simplified such that many of the components utilized to facilitate communications are not shown. One skilled in the relevant art will appreciate that such components can be utilized and that additional interactions would accordingly occur without departing from the spirit and scope of the present disclosure.

FIG. 4 is a block diagram illustrative of a hypoglycemic unawareness identification environment 400 illustrating a process for determining a patient's level of hypo-unawareness, in accordance with an example embodiment of the present disclosure. The process begins with a HUDTS 405 receiving glucose data. The glucose data can be received from at least one of a glucose measurement device (e.g., device 125 of FIG. 1) and input received from a user interface of a patient device 420. In response to receiving the glucose data, the HUDTS 405 identifies prompts to send in a query message to the patient device 420. The HUDTS 405 identifies the prompts by accessing a data structure that correlates glucose concentrations with one or more prompts. Example prompts can be can be categorized in at least one of the following categories: symptoms recognized by the patient, attempted self-treatment methods used by the patient, and the patient's perceived cause of becoming hypoglycemic. For example, prompts can ask the patient for information regarding autonomic symptoms e.g., pounding heart, shakiness, and sweatiness) and neuroglypenic symptoms (e.g., light headiness, confusion, and lack of coordination).

It should be noted that the query can include any number of prompts. A number of prompts can be selected based on a prompt fatigue metric associated with the patient. The prompt fatigue metric can be a threshold value that is based upon at least a history of patient responsiveness to previous prompts. In another example, the number can be adjusted by the patient or a clinician.

Once the HUDTS 405 identifies the prompts, the HUDTS 405, transmits the query to the patient device 420 via network 410. In response to receiving the query, the patient device issues the prompts via, for example, a user interface to the patient. The patient enters responses to the prompts via the user interface. Once the patient responds to the prompts, the patient device 420 transmits the responses to the HUDTS 405 via the network 410. The HUDTS 405 received the patient responses to the prompts and analyzes the responses. In an example, the HUDTS 405 analyzes the responses using a data structure that correlates the patient's responses to prompts to the glucose concentration and the severity of the patient's symptoms to derive a score indicating the degree of hypo-unawareness. Based on the analysis, the HUDTS 405 determines the patient's level of hypo-unawareness and generates a hypo-report.

The HUDTS 405 transmits the hypo-report to an EMR system 435 via the network 410. The EMR system 435 receives the hypo-report and stores the report in a file repository associated with the patient. A clinician is then able to access the report via a health care provider device 430.

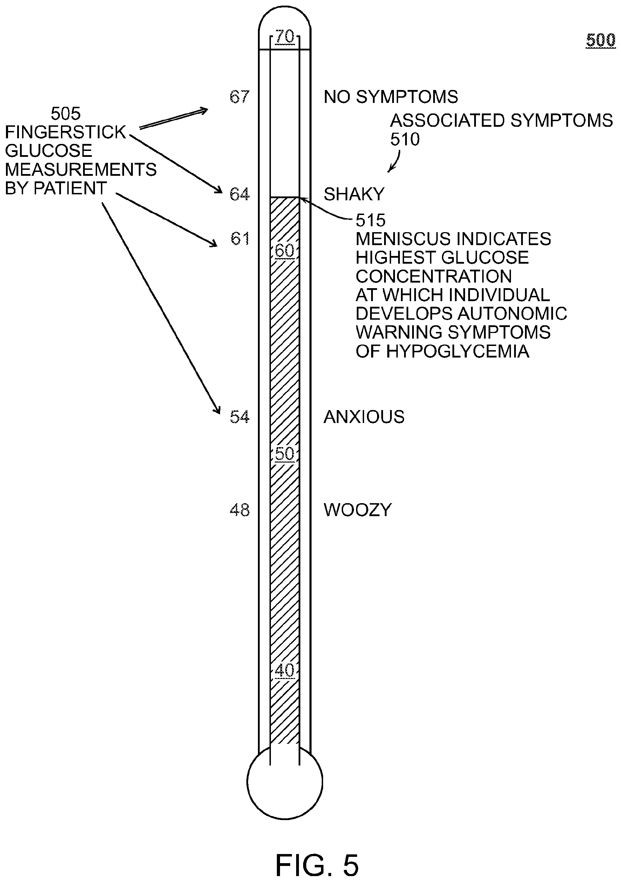

FIG. 5 is a diagram illustrating a hypo-report 500, in accordance with an example embodiment of the present disclosure. The hypo-report 500 is represented as a thermometer that correlates a blood glucose level 505 with symptoms 510 associated with the blood glucose concentration Cmg/dl). It should be noted that although the hypo-report 500 is represented as a thermometer and depiction can be used that correlates glucose concentrations with respective symptoms. The hypo-report 500 can be displayed on a PDA or other suitable device that a patient can readily understand and with which the patient can easily interact.

Symptoms can be categorized as either autonomic or neuroglycopenic. Example autonomic symptoms can include at least one of the following: pounding heart, shakiness, and sweatiness. Example neuroglycopenic symptoms can include at least one of the following: light-headiness, lack of coordination, and confusion.

The example shown in FIG. 5 illustrates a patient having a glucose concentration of 64 *mg*/*dl.* According to the thermometer 500, such a concentration should result in the patient having shaky symptoms. The highest glucose concentration in the hypoglycemia range that a patient develops symptoms indicates the threshold at which the patient recognizes hypoglycemia. If this concentration is:s 60 mg/dL this would indicate that the patient has hypo-unawareness. With more severe hypo-unawareness this threshold for recognition of hypoglycemia would be lower.

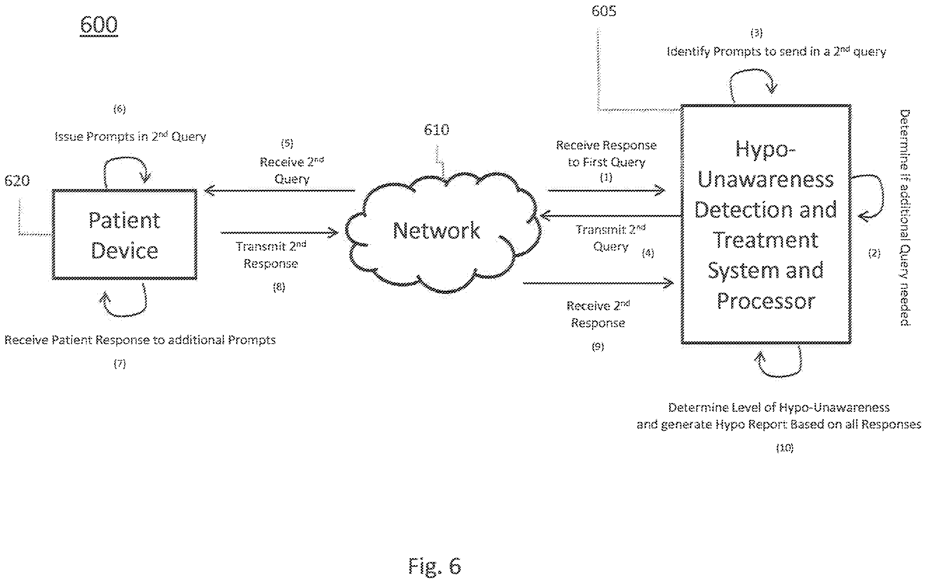

FIG. 6 is a block diagram illustrative of a hypoglycemic unawareness identification environment 600 illustrating a process for issuing a second query based on patient responses to a first query, in accordance with an example embodiment of the present disclosure.

The process begins with a HUDTS 605 receiving a patient's response to prompts issued in a first query. In response to receive the patient's response to the prompts, the HUDTS 605 determines if additional input from the patient is needed to determine the patient's level of hypo-unawareness. In particular, the HUDTS 605 analyzes the responses to assess whether a determination regarding the patient's level of hypo-unawareness can be made. If a determination cannot be made, the HUDTS 605 identifies prompts that have a high degree of probability of receiving responses to enable the HUDTS 605 to determine the patient's level of hypo-unawareness.

Once the HUDTS 605 identifies the prompts, the HUDTS 605 transmits a second query to the patient device 610 that includes the identified prompts. Upon receipt of the second query, the patient device 610 issues the prompts the patient. In response to receiving the patient's responses, the patient device 610 transmits the second responses to the HUDTS 605. The HUDTS 605 receives the second responses and determines the patient's level of hypo-unawareness based on all responses to the prompts that have been issued. It should be noted that the process can have additional iterations. In particular, additional prompts can be issued by the HUDTS 605 until responses are received that enable the HUDTS 605 to determine the patient's level of hypo-unawareness.

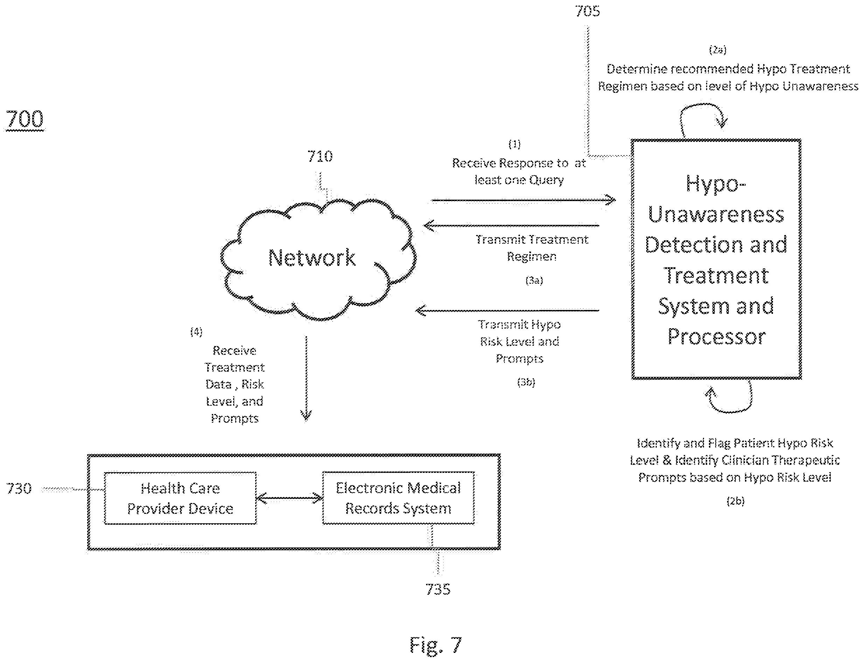

FIG. 7 is a block diagram illustrative of a hypoglycemic unawareness identification environment 700 illustrating a process for providing a clinician with treatment options, in accordance with an example embodiment of the present disclosure. The process begins with a HUDTS 705 receiving patient responses to prompts provided to a patient. Once the HUDTS 705 received the responses, the HUDTS 705 determines a hypoglycemia minimization recommendations based a patient's determined level of hypo-unawareness. In particular, the HUDTS 705 can include a data structure that correlates a patient's level of hypo-unawareness with certain treatment options. In addition, the HUDTS 705 can flag the patient as being at risk for frequently reaching hypoglycemic state based on the patient's determined level of hypo-unawareness. The HUDTS 705 then identifies clinician therapeutic prompts that are used by a clinician to determine the patient's current diabetes management program can be optimized to minimize risk for hypoglycemia.

The HUDTS 705 the transmits the identified treatment regimen and clinician therapeutic prompts to an EMR system 735, which stored the data in a data storage device. The clinician can access the transmitted data via a health care provider device 730. In practice, a clinician is able to access the data (on demand) during a patient's appointment. The clinician can review the treatment recommendations including need to eat at specific times, starting insulin pump therapy and/or continuous glucose monitoring.

The above-described systems and methods can be implemented in digital electronic circuitry, in computer hardware, firmware, and/or software. The implementation can be as a computer program product. The implementation can, for example, be in a non-transitory machine-readable storage device, for execution by, or to control the operation of, data processing apparatus. The implementation can, for example, be a programmable processor, a computer, and/or multiple computers.

A computer program can be written in any form of programming language, including compiled and/or interpreted languages, and the computer program can be deployed in any form, including as a stand-alone program or as a subroutine, element, and/or other unit suitable for use in a computing environment. A computer program can be deployed to be executed on one computer or on multiple computers at one site.

Method steps can be performed by one or more programmable processors executing a computer program to perform functions of the invention by operating on input data and generating output. Method steps can also be performed by and an apparatus can be implemented as special purpose logic circuitry. The circuitry can, for example, be a FPGA (field programmable gate array) and/or an ASIC (application-specific integrated circuit). Subroutines and software agents can refer to portions of the computer program, the processor, the special circuitry, software, and/or hardware that implement that functionality.

Processors suitable for the execution of a computer program include, by way of example, both general and special purpose microprocessors, and anyone or more processors of any kind of digital computer. Generally, a processor receives instructions and data from a read-only memory or a random access memory or both. The essential elements of a computer are a processor for executing instructions and one or more memory devices for storing instructions and data. Generally, a computer can include, can be operatively coupled to receive data from and/or transfer data to one or more mass storage devices for storing data (e.g., magnetic, magneto-optical disks, or optical disks).

Data transmission and instructions can also occur over a communications network. Information carriers suitable for embodying computer program instructions and data include all forms of non-volatile memory, including by way of example semiconductor memory devices. The information carriers can, for example, be EPROM, EEPROM, flash memory devices, magnetic disks, internal hard disks, removable disks, magneto-optical disks, CD-ROM, and/or DVD-ROM disks. The processor and the memory can be supplemented by, and/or incorporated in special purpose logic circuitry.

To provide for interaction with a user, the above described techniques can be implemented on a computer having a display device. The display device can, for example, be a cathode ray tube (CRT) and/or a liquid crystal display (LCD) monitor. The interaction with a user can, for example, be a display of information to the user and a keyboard and a pointing device (e.g., a mouse or a trackball) by which the user can provide input to the computer (e.g., interact with a user interface element). Other kinds of devices can be used to provide for interaction with a user. Other devices can, for example, be feedback provided to the user in any form of sensory feedback (e.g., visual feedback, auditory Feedback, or tactile feedback). Input from the user can, for example, be received in any form, including acoustic, speech, and/or tactile input.

The above described techniques can be implemented in a distributed computing system that includes a back-end component. The back-end component can, for example, be a data server, a middleware component, and/or an application server. The above described techniques can be implemented in a distributing computing system that includes a front-end component. The front-end component can, for example, be a client computer having a graphical user interface, a Web browser through which a user can interact with an example implementation, and/or other graphical user interfaces for a transmitting device. The components of the system can be interconnected by any form or medium of digital data communication (e.g., a communication network). Examples of communication networks include a local area network (LAN), a wide area network (WAN), the Internet, wired networks, and/or wireless networks.

The system can include clients and servers. A client and a server are generally remote from each other and typically interact through a communication network. The relationship of client and server arises by virtue of computer programs running on the respective computers and having a client-server relationship to each other.

Packet-based networks can include, for example, the Internet, a carrier internet protocol (IP) network (e.g., local area network (LAN), wide area network (WAN) campus area network (CAN), metropolitan area network (MAN), home area network (HAN», a private **IP** network, an **IP** private branch exchange (IPBX), a wireless network (e.g., radio access network (RAN), 802.11 network, 802.16 network, general packet radio service (GPRS) network, HiperLAN), and/or other packet-based networks. Circuit-based networks can include, for example, the public switched telephone network (PSTN), a private branch exchange (PBX), a wireless network (e.g., RAN, Bluetooth, code-division multiple access (CDMA) network, time division multiple access (TDMA) network, global system for mobile communications (GSM) network), and/or other for mobile communications (GSM) network), and/or other circuit-based networks.

The transmitting device can include, for example, a computer, a computer with a browser device, a telephone, an IP phone, a mobile device (e.g., cellular phone, personal digital assistant (PDA) device, laptop computer, electronic mail device), and/or other communication devices. The browser device includes, for example, a computer (e.g., desktop computer, laptop computer) with a World Wide Web browser (e.g., Microsoft® Internet Explorer® available from Microsoft Corporation, Mozilla® Firefox available from Mozilla Corporation). The mobile computing device includes, for example, an iPhone®.

## Claims

1. A method of determining a level of hypoglycemic unawareness displayed by a patient, comprising:
maintaining, in a data storage device in communication with one or more processors, a data structure including one or more glucose concentrations correlated to one or more subject prompts;
receiving, by the one or more processors, a glucose concentration;
determining, by the one or more processors, a query based upon the received glucose concentration and the corresponding one or more subject
prompts from the data structure;
transmitting, to a user interface device in communication with the one or more processors, the query;
receiving, by the one or more processors, a patient response to the transmitted query; and
determining, by the one or more processors, a level of hypoglycemic unawareness of the patient based at least upon the patient response.

2. The method of claim 1, wherein the query includes one or more prompts and wherein the patient response includes one or more answers responsive to respective ones of the one or more prompts, and wherein the method
further comprises:
determining, by the one or more processors, another query including one or more additional prompts based on the one or more answers responsive to respective ones of the one or more prompts;
transmitting, to a user interface device in communication with the one or more processors, the other query; and
determining a level of hypoglycemic unawareness of the patient based at least upon the patient response to the one or more prompts and the one or more additional prompts.

3. The method of claim 1 wherein determining the query includes determining, by the one or more processors, a number of prompts to include in the query based on a prompt fatigue metric associated with the patient.

4. The method of claim 3 wherein the prompt fatigue metric is based on a history of patient responsiveness to prompts.

5. The method of claim 1 further comprising, by the one or more processors providing a clinician with therapeutic recommendations for restoring hypo awareness to the patient based on the determined level of hypoglycemic unawareness of the patient.

6. The method of claim 1 further comprising, by the one or more processors, providing a clinician with therapeutic intervention prompts based on an indication of a patient's risk for being hypo unaware.

7. The method of claim 6, wherein the indication of a patient's risk for being hypo unaware is based on a previous determination of a threshold level of hypoglycemic unawareness of the patient.

8. A system for determining a level of hypoglycemic unawareness displayed by a patient, the system comprising:
a data storage device configured to maintain a data structure including one or more glucose concentrations correlated to one or more subject prompts;
one or more processors in communication with the data storage device, the one or more processors configured to:
receive a glucose concentration;
determine a query based upon the received glucose concentration and the corresponding one or more subject prompts from the data structure;
transmit the query to a user interface device in communication with the one or more processors;
receive a patient response to the transmitted query; and
determine a level of hypoglycemic unawareness of the patient based at least upon the patient response.

9. The apparatus of claim 8, wherein the query includes one or more prompts and wherein the patient response includes one or more answers responsive to respective ones of the one or more prompts, and wherein the one or more processors are further configured to:
determine another query including one or more additional prompts based on the one or more answers responsive to respective ones of the one or more prompts;
transmit, to a user interface device in communication with the one or more processors, the other query; and
determine a level of hypoglycemic unawareness of the patient based at least upon the patient response to the one or more prompts and the one or more additional prompts.

10. The apparatus of claim 8 wherein the one or more processors are further configured to determine a number of prompts to include in the query based on a prompt fatigue metric associated with the patient.

11. The apparatus of claim 10 wherein the prompt fatigue metric is based on a history of patient responsiveness to prompts.

12. The apparatus of claim 8 wherein the one or more processors are further configured to provide a clinician with therapeutic recommendations to restore hypo awareness to the patient based on the determined level of hypoglycemic unawareness of the patient.

13. The apparatus of claim 8 wherein the one or more processors are further configured to provide a clinician with therapeutic intervention prompts based on an indication of a patient's risk for being hypo and hypo unaware.

14. The apparatus of claim 13, wherein the indication of a patient's risk for being hypo unaware is based on a previous determination of a threshold level of hypoglycemic unawareness of the patient.

15. A non-transitory computer readable medium having computer readable program codes embodied thereon for determining a level of hypoglycemic unawareness instructions that, when executed by a processor, cause the processor to:
maintain a data structure including one or more glucose concentration correlated to one or more subject prompts;
receive a glucose concentration;
determine a query based upon the received glucose concentration and the corresponding one or more subject prompts from the data structure;
transmit the query to a user interface device;
receive a patient response to the transmitted query; and
determine a level of hypoglycemic unawareness of the patient based at least upon the patient response.

## Patentansprüche

1. Verfahren zum Bestimmen des von einem Patienten angezeigten Grades an Hypoglykämie-Wahrnehmungsstörung, das Folgendes umfasst:
Aufrechterhalten einer Datenstruktur, die eine oder mehrere Glukosekonzentrationen enthält, welche mit einer oder mehreren Subjekt-Eingabeaufforderungen korreliert sind, in einer Datenspeichervorrichtung in Verbindung mit einem oder mehreren Prozessoren;
Empfangen einer Glukosekonzentration durch den einen oder die mehreren Prozessoren;
Bestimmen einer Abfrage auf Grundlage der empfangenen Glukosekonzentration und den einen oder die mehreren entsprechenden Subjekt-Eingabeaufforderungen von der Datenstruktur durch den einen oder die mehreren Prozessoren;
Übertragen der Abfrage zu einer Benutzerschnittstellenvorrichtung in Verbindung mit dem einen oder den mehreren Prozessoren;
Empfangen einer Patientenreaktion auf die übertragene Abfrage durch den einen oder die mehreren Prozessoren; und
Bestimmen des Grades an Hypoglykämie-Wahrnehmungsstörung des Patienten auf Grundlage wenigstens der Patientenreaktion durch den einen oder die mehreren Prozessoren.

2. Verfahren nach Anspruch 1, wobei die Abfrage eine oder mehrere Eingabeaufforderungen umfasst und wobei die Patientenreaktion eine oder mehrere Antworten umfasst, die auf die jeweiligen einen der einen oder mehreren Eingabeaufforderungen reagieren, und wobei das Verfahren ferner Folgendes umfasst:
Bestimmen einer weiteren Abfrage, die eine oder mehrere zusätzliche Eingabeaufforderungen enthält, die auf der einen oder den mehreren Antworten basieren, welche auf die jeweiligen einen der einen oder mehreren Eingabeaufforderungen reagieren, durch den einen oder die mehreren Prozessoren;
Übertragen der anderen Abfrage zu einer Benutzerschnittstellenvorrichtung in Verbindung mit dem einen oder den mehreren Prozessoren; und
Bestimmen des Grades an Hypoglykämie-Wahrnehmungsstörung des Patienten auf Grundlage wenigstens der Patientenreaktion auf die eine oder mehreren Eingabeaufforderungen und die eine oder mehreren zusätzlichen Eingabeaufforderungen.

3. Verfahren nach Anspruch 1, wobei das Bestimmen der Abfrage das Bestimmen einer Anzahl von Eingabeaufforderungen zur Aufnahme in die Abfrage auf Grundlage einer dem Patienten zugehörigen Metrik für die Eingabeaufforderungsermüdung durch den einen oder die mehreren Prozessoren enthält.

4. Verfahren nach Anspruch 3, wobei die Metrik für Eingabeaufforderungsermüdung auf einer früheren Reaktionsfähigkeit des Patienten auf Eingabeaufforderungen basiert.

5. Verfahren nach Anspruch 1, das ferner umfasst, durch den einen oder die mehreren Prozessoren einem Kliniker therapeutische Empfehlungen zum Wiederherstellen der Hypoglykämie-Wahrnehmung des Patienten auf Grundlage des bestimmten Grades an Hypoglykämie-Wahrnehmungsstörung des Patienten bereitzustellen.

6. Verfahren nach Anspruch 1, das ferner umfasst, durch den einen oder die mehreren Prozessoren einem Kliniker therapeutische Interventions-Eingabeaufforderungen auf Grundlage eines Hinweises auf das Risiko des Patienten, die Hypoglykämie nicht wahrzunehmen, bereitzustellen.

7. Verfahren nach Anspruch 6, wobei der Hinweis auf das Risiko eines Patienten, die Hypoglykämie nicht wahrzunehmen, auf einer früheren Bestimmung eines Schwellenwertes der Hypoglykämie-Wahrnehmungsstörung des Patienten basiert.

8. System zum Bestimmen des von einem Patienten angezeigten Grades an Hypoglykämie-Wahrnehmungsstörung, wobei das System Folgendes umfasst:
eine Datenspeichervorrichtung, so konfiguriert, dass sie eine Datenstruktur aufrechterhält, die eine oder mehrere Glukosekonzentrationen enthält, welche mit einer oder mehreren Subjekt-Eingabeaufforderungen korreliert sind;
ein oder mehrere Prozessoren in Verbindung mit der Datenspeichervorrichtung, wobei der eine oder die mehreren Prozessoren so konfiguriert sind, dass sie:
eine Glukosekonzentration empfangen;
eine Abfrage auf Grundlage der von der Datenstruktur empfangenen Glukosekonzentration und der entsprechenden einen oder mehreren Subjekt-Eingabeaufforderungen bestimmen;
die Abfrage an eine Benutzerschnittstellenvorrichtung in Verbindung mit dem einen oder mehreren Prozessoren übertragen;
eine Patientenreaktion auf die übertragene Abfrage empfangen; und
den Grad an Hypoglykämie-Wahrnehmungsstörung des Patienten auf Grundlage wenigstens der Patientenreaktion bestimmen.

9. Vorrichtung nach Anspruch 8, wobei die Abfrage eine oder mehrere Eingabeaufforderungen enthält und wobei die Patientenreaktion eine oder mehrere Antworten, die auf die jeweiligen einen der einen oder mehreren Eingabeaufforderungen reagieren, enthält und wobei der eine oder die mehreren Prozessoren ferner so konfiguriert sind, dass sie:
eine weitere Abfrage, die eine oder mehrere zusätzliche Eingabeaufforderungen auf Grundlage der einen oder mehreren Antworten, die auf die jeweiligen einen der einen oder mehreren Eingabeaufforderungen reagieren, bestimmen;
die andere Abfrage zu einer Benutzerschnittstellenvorrichtung in Verbindung mit dem einen oder mehreren Prozessoren übertragen; und
den von dem Patienten angezeigten Grad an Hypoglykämie-Wahrnehmungsstörung auf Grundlage wenigstens der Patientenreaktion auf die eine oder mehreren Eingabeaufforderungen und auf die eine oder mehreren zusätzlichen Eingabeaufforderungen bestimmen.

10. Vorrichtung nach Anspruch 8, wobei der eine oder die mehreren Prozessoren ferner so konfiguriert sind, dass sie eine Anzahl von Eingabeaufforderungen zur Aufnahme in die Abfrage auf Grundlage einer dem Patienten zugehörigen Metrik für die Eingabeaufforderungsermüdung bestimmen.

11. Vorrichtung nach Anspruch 10, wobei die Metrik für die Eingabeaufforderungsermüdung auf einer früheren Reaktionsfähigkeit des Patienten auf Eingabeaufforderungen basiert.

12. Vorrichtung nach Anspruch 8, wobei der eine oder die mehreren Prozessoren ferner so konfiguriert sind, dass sie einem Kliniker therapeutische Empfehlungen für den Patienten zum Wiederherstellen der Hypoglykämie-Wahrnehmung auf Grundlage des bestimmten Grades an Hypoglykämie-Wahrnehmungsstörung des Patienten bereitstellen.

13. Vorrichtung nach Anspruch 8, wobei der eine oder die mehreren Prozessoren ferner so konfiguriert sind, dass sie einem Kliniker auf Grundlage eines Hinweises auf das Risiko des Patienten, hypoglykämisch zu sein und die Hypoglykämie nicht wahrzunehmen, therapeutische Interventions-Eingabeaufforderungen bereitstellen.

14. Vorrichtung nach Anspruch 13, wobei der Hinweis auf das Risiko des Patienten, die Hypoglykämie nicht wahrzunehmen, auf einer früheren Bestimmung eines Schwellenwertes der Hypoglykämie-Wahrnehmungsstörung des Patienten basiert.

15. Nicht-transitorisches computerlesbares Medium mit darauf gespeicherten computerlesbaren Programmcodes mit Anweisungen zum Bestimmen eines Grades an Hypoglykämie-Wahrnehmungsstörung, die bei Ausführung durch den Prozessor den Prozessor veranlassen:
eine Datenstruktur, die eine oder mehrere Glukosekonzentrationen enthält, die mit einer oder mehreren Subjekt-Eingabeaufforderungen korreliert sind, aufrechtzuerhalten;
eine Glukosekonzentration zu empfangen;
eine Abfrage auf Grundlage der empfangenen Glukosekonzentration und den entsprechenden einen oder mehreren Subjekt-Eingabeaufforderungen von der Datenstruktur zu bestimmen;
die Abfrage zu einer Benutzerschnittstellenvorrichtung zu übertragen;
eine Patientenreaktion auf die übertragene Abfrage zu empfangen; und
den Grad an Hypoglykämie-Wahrnehmungsstörung des Patienten auf Grundlage wenigstens der Patientenreaktion zu bestimmen.

## Revendications

1. Procédé de détermination de niveau d'insensibilité à l'hypoglycémie affichée par un patient, comprenant :
le maintien, dans un dispositif de stockage de données en communication avec un ou plusieurs processeurs, d'une structure de données comprenant une ou plusieurs concentrations de glucose en corrélation avec une ou plusieurs invites de patient ;
la réception, par les un ou plusieurs processeurs, d'une concentration de glucose ;
la détermination, par les un ou plusieurs processeurs, d'une requête basée sur la concentration de glucose reçue et des une ou plusieurs invites de patient correspondantes à partir de la structure de données ;
la transmission, à un dispositif d'interface utilisateur en communication avec les un ou plusieurs processeurs, de la requête ;
la réception, par les un ou plusieurs processeurs, d'une réponse du patient à la requête transmise ; et
la détermination, par les un ou plusieurs processeurs, d'un niveau d'insensibilité à l'hypoglycémie du patient au moins sur la base de la réponse du patient.

2. Procédé selon la revendication 1, dans lequel la requête comprend une ou plusieurs invites et dans lequel la réponse du patient comprend une ou plusieurs réponses en réponse à des invites respectives parmi les une ou plusieurs invites, et dans lequel le procédé comprend en outre :
la détermination, par les un ou plusieurs processeurs, d'une autre requête comprenant une ou plusieurs invites supplémentaires basées sur les une ou plusieurs réponses en réponse à des invites respectives parmi les une ou plusieurs invites ;
la transmission, à un dispositif d'interface utilisateur en communication avec les un ou plusieurs processeurs, de l'autre requête ; et
la détermination d'un niveau d'insensibilité à l'hypoglycémie du patient au moins sur la base de la réponse du patient aux une ou plusieurs invites et aux une ou plusieurs invites supplémentaires.

3. Procédé selon la revendication 1, dans lequel la détermination de la requête comprend la détermination, par les un ou plusieurs processeurs, d'un nombre d'invites à inclure dans la requête sur la base d'un indicateur de fatigue d'invite associé au patient.

4. Procédé selon la revendication 3, dans lequel l'indicateur de fatigue d'invite est basé sur un historique de la réactivité d'un patient aux invites.

5. Procédé selon la revendication 1 comprenant en outre, par les un ou plusieurs processeurs, la fourniture à un médecin de recommandations thérapeutiques pour restaurer l'hyposensibilité du patient sur la base d'un niveau déterminé d'insensibilité à l'hypoglycémie du patient.

6. Procédé selon la revendication 1 comprenant en outre, par les un ou plusieurs processeurs, la fourniture à un médecin d'invites d'intervention thérapeutique sur la base d'une indication d'un risque qu'un patient soit hypo-insensible.

7. Procédé selon la revendication 6, dans lequel l'indication d'un risque qu'un patient soit hypo-insensible est basée sur une détermination préalable d'un niveau seuil d'insensibilité à l'hypoglycémie du patient.

8. Système de détermination de niveau d'insensibilité à l'hypoglycémie affichée par un patient, le système comprenant :
un dispositif de stockage de données conçu pour maintenir une structure de données comprenant une ou plusieurs concentrations de glucose en corrélation avec une ou plusieurs invites de patient ;
un ou plusieurs processeurs en communication avec le dispositif de stockage de données, les un ou plusieurs processeurs étant conçus pour :
recevoir une concentration de glucose ;
déterminer une requête sur la base de la concentration de glucose reçue et des une ou plusieurs invites de patient correspondantes à partir de la structure de données ;
transmettre la requête à un dispositif d'interface utilisateur en communication avec les un ou plusieurs processeurs ;
recevoir une réponse du patient à la requête transmise ; et
déterminer un niveau d'insensibilité à l'hypoglycémie du patient au moins sur la base de la réponse du patient.

9. Appareil selon la revendication 8, dans lequel la requête comprend une ou plusieurs invites et dans lequel la réponse du patient comprend une ou plusieurs réponses en réponse à des invites respectives parmi les une ou plusieurs invites, et dans lequel les un ou plusieurs processeurs sont en outre conçus pour :
déterminer une autre requête comprenant une ou plusieurs invites supplémentaires sur la base des une ou plusieurs réponses en réponse à des invites respectives parmi les une ou plusieurs invites ;
transmettre, à un dispositif d'interface utilisateur en communication avec les un ou plusieurs processeurs, l'autre requête ; et
déterminer un niveau d'insensibilité à l'hypoglycémie du patient au moins sur la base de la réponse du patient aux une ou plusieurs invites et aux une ou plusieurs invites supplémentaires.

10. Appareil selon la revendication 8, dans lequel les un ou plusieurs processeurs sont en outre conçus pour déterminer un nombre d'invites à inclure dans la requête sur la base d'un indicateur de fatigue d'invite associé au patient.

11. Appareil selon la revendication 10, dans lequel l'indicateur de fatigue d'invite est basé sur un historique de la réactivité d'un patient aux invites.

12. Appareil selon la revendication 8, dans lequel les un ou plusieurs processeurs sont en outre conçus pour fournir à un médecin des recommandations thérapeutiques pour restaurer l'hypo-sensibilité du patient sur la base d'un niveau déterminé d'insensibilité à l'hypoglycémie du patient.

13. Appareil selon la revendication 8, dans lequel les un ou plusieurs processeurs sont en outre conçus pour fournir à un médecin des invites d'intervention thérapeutique sur la base d'une indication d'un risque qu'un patient soit hypo et hypo-insensible.

14. Appareil selon la revendication 13, dans lequel l'indication d'un risque qu'un patient soit hypo-insensible est basée sur une détermination préalable d'un niveau seuil d'insensibilité à l'hypoglycémie du patient.

15. Support non transitoire lisible par ordinateur ayant des codes de programme lisibles par ordinateur intégrés à celui-ci, destiné à déterminer un niveau d'instructions d'insensibilité à l'hypoglycémie qui, lorsqu'elles sont exécutées par un processeur, amènent le processeur à :
maintenir une structure de données comprenant une ou plusieurs concentrations de glucose en corrélation avec une ou plusieurs invites de patient ;
recevoir une concentration de glucose ;
déterminer une requête sur la base de la concentration de glucose reçue et des une ou plusieurs invites de patient correspondantes à partir de la structure de données ;
transmettre la requête à un dispositif d'interface utilisateur ;
recevoir une réponse du patient à la requête transmise ; et
déterminer un niveau d'insensibilité à l'hypoglycémie du patient au moins sur la base de la réponse du patient.
